# EUROPEAN PATENT APPLICATION

(11) **EP 1 652 859 A1**
(43) Date of publication of application: **03.05.2006**
(21) Application number: 04025936.8
(22) Date of filing: 02.11.2004
(51) Int. Cl.: C07K 16/06, C07K 16/00, A61K 39/00, A61K 39/395

(54) **Immunoglobulin fractions**

(71) Applicant: ZLB Behring AG, 3014 Bern (CH)
(72) Inventor: Miescher, Sylvia, 3004 Bern (CH); Bolli, Reinhard Franz, 3076 Worb (CH)
(74) Representative: Pfeil, Hugo

(57) **Abstract**

The present invention relates to novel pharmaceutical compositions comprising fractions of human immunoglobulin preparations, novel applications of these compositions and a method for manufacturing the compositions.

## Description

The present invention relates to novel pharmaceutical compositions comprising fractions of human immunoglobulin preparations, novel applications of these compositions and a method for manufacturing the compositions.

Intravenous immunoglobulin (IVIG) preparations are derived from pooled plasma of thousands of healthy donors (current pool sizes range from 1000 - 60,000) and contain both immune and natural antibodies (NAbs) reflecting the cumulative antigen experience of the donor population. IVIG thus contains a large spectrum of so called "immune antibodies" specificities directed against pathogens and foreign antigens as well as NAbs reacting with self/autoantigens. NAbs are so defined as they are generated in the absence of deliberate immunization and independently of exposure to foreign antigens (56). Indeed a long list of antibodies to self antigens has been detected and even purified by affinity chromatography from IVIG preparations (2, 3, 4, 5, 9, 16, 55). These NAbs are often polyreactive, can bind to pathogens and by tagging and clearing senescent or altered molecules and cells from the circulation are important in maintaining immunohomeostasis (57). Further, it has been proposed that these NAbs recognise a conserved subset of immunodominant antigens referred to as the immunological homunculus that is essential for maintaining tolerance to self (8, 15, 21). However, a more precise characterisation of these antibodies, their properties and their potential presence in a sub-fraction of IVIG has not been defined partly due to the paucity of knowledge on the relevant target antigens and antibodies.

Current therapeutic applications of IVIG cover 2 major categories: 1) antibody replacement in primary and secondary antibody deficiencies (18, 27, 43, 48, 52) immunomodulation in patients with systemic inflammatory and autoimmune diseases (10, 11, 14, 17, 19, 22, 23, 24, 25, 26; 27; 28; 39, 40, 41, 43; 45, 47, 53, 58, 61, 62, 63) . While IVIG has shown clinical efficacy in the antibody deficiencies and in some autoimmune and inflammatory diseases [(a few egs. Immune Thrombocytopaenic Purpura (ITP), Guillain Barré Syndrome (GBS), Kawasaki disease, Chronic Inflammatory Demyelineating Polyneuropathy (CIDP)] there are many examples of unconfirmed/equivocal clinical benefits in other autoimmune and systemic inflammatory conditions often due to lack of statistically significant controlled trials.

The mode of action of IVIG is complex involving modulation of the expression and function of Fc receptors, interference with complement activation and the cytokine network, modulation of activation, differentiation and effector functions in many different cell types. The idiotype network has been proposed as an essential network involved in immune homeostasis with imbalances eventually leading to overt autoimmune disease. Many of the current therapeutic indications for IVIG are based on counterbalancing, neutralising or blocking pathological autoimmune antibodies and thus restoring a healthy idiotype network (6, 7, 49, 64). Recent reports have also shown direct effects on monocytes, dendritic cells, endothelial and both T and B lymphocytes. This complexity reflects the essential functions of circulating antibodies in maintaining homeostasis in healthy individuals (34, 36, 37, 42, 44, 46, 50, 51, 54).

There are many reports in the literature demonstrating the decrease in immune reactivity with age, so called immunosenescence (12, 13, 29-32, 38). However it appears that there is a life long stability of natural IgG antibodies reacting with self antigens compared to an age dependent diversification of IgG antibody repertoires to foreign antigens with increasing age (1, 60).

IVIG consists of intact IgG molecules with an IgG isotype distribution corresponding to that of normal human serum and trace amounts of IgA.

Depending on the formulation, IVIG preparations contain variable amounts of monomeric and dimeric IgG. Monomers represent the monomeric IgG (ca. mol.weight 150,000) while dimers represent 2 IgG molecules which from previous data can be orientated as classical idiotype anti-idiotype pairs (20, 33, 35, 65).

EP-A-1394183 discloses a method to purify autoantibodies from IVIG preparations using affinity chromatography on a ligand, e.g. a mixture of proteins present in human serum other than immunoglobulin bound to a solid support.

U.S. Patent 6,231,856 discloses human antibody compositions comprising substantially purified and concentrated anti-idiotypic antibodies or antigen-binding fragments thereof for the treatment of antibody-based autoimmune diseases. The antibody compositions are obtained by affinity chromatography.

According to the present invention, it was found that a size fractionation of a human immunoglobulin preparation gives a fraction of dimeric antibodies and a fraction of monomeric antibodies which have different characteristics. The dimeric fraction shows an increased reactivity to antigens, e.g. self-antigens and/or exoantigens. Further, the dimeric fraction contains antibodies with higher affinity to target antigens and thus may have a higher biological activity, e.g. neutralizing activity in the case of anti-bacterial, antitoxin or anti-viral activities. In contrast thereto, the monomeric fraction may contain lower affinity antibodies, e.g. natural antibodies, i.e. the basic repertoire from which high affinity antibodies develop.

In a first aspect, the present invention relates to a pharmaceutical composition comprising a fraction of human antibodies or antigen-binding fragments thereof from a heterogenous human donor population wherein the fraction originates from the dimeric human antibodies.

In a further aspect, the present invention relates to a pharmaceutical composition comprising a fraction of human antibodies or antigen-binding fragments thereof from a heterogenous human donor population wherein the fraction originates from the monomeric human antibodies.

The fractions may be obtained from any preparation comprising polyclonal human antibodies preparation from a plurality of donors. Preferably, the fractions are obtained from an IVIG preparation. In this context, an IVIG preparation is a human immunoglobulin preparation which may be derived from pooled plasma of a plurality, e.g. 1,000-100,000 of blood donors. Antigen-binding fragments of the antibodies may be obtained by known methods, e.g. by proteolytic digestion. Preferred antibody fragments are Fab fragments or F(ab')₂ fragments.

The monomeric or dimeric fractions may be obtained by size fractionation of human antibody preparations. The size fractionation may comprise size exclusion separation, gel filtration, ultrafiltration, diafiltration and/or other gel or membrane separation methods. The monomeric fraction may be obtained by recovering antibody monomers with an apparent molecular weight of about 150 kDa and the dimeric fraction may be obtained by recovering antibody dimers with an apparent molecular weight of about 300 kDa.

Preferably, the size fractionation comprises size exclusion chromatography, e.g. on a Superose 6 or a Sephacryl S-300 column.

The purity of the monomeric and dimeric fractions obtained by size fractionation may be measured by analytical HPLC, e.g. size exclusion chromatography on a TSK G 3000 SWXL column.

The purity of dimeric antibody fraction is usually at least 75%, preferably at least 80% and more preferably at least 85% as determined by analytical HPLC wherein the analysis is preferably carried out immediately after size fractionation.

The purity of monomeric antibody fraction is usually at least 85%, preferably at least 90% and more preferably at least 95% as determined by analytical HPLC, wherein the analysis is preferably carried out immediately after size fractionation.

The dimeric antibody fraction is preferably characterized by an increased reactivity to exoantigens such as tetanus toxoid and/or respiratory syncytial virus (RSV) and/or autoantigens such as red blood cell ghosts and/or Hep-2 epithelial cells. More particularly, the dimeric fraction may have an at least two times increased specific activity against tetanus toxoid and/or respiratory syncytial virus compared to an unfractionated IVIG preparation as determined in the Examples section. Further, the dimeric fraction may have an at least two times increased reactivity towards an M virulence protein of S.pyogenes and/or Hep-2 epithelial cells compared to an unfractionated IVIG preparation as determined in the Examples section.

The pharmaceutical composition of the dimeric antibody fraction may comprise antibodies in a dimeric form or in a dissociated or (re)monomerised form. Preferably the antibodies are in a dissociated or (re)monomerised form, wherein the content of dimers is 10% or less, more preferably 5% or less. Monomerisation may be carried out by adjusting an acidic pH, e.g. an pH in the range of about 3 to about 5, preferably about 3.5 to about 4.5, more preferably a pH of about 4.0. The pH may be adjusted by any pharmaceutically acceptable acidic buffering agent, e.g. acetic acid. The monomerised preparation of dimeric antibodies is preferably dynamically stable and substantially does not reconvert to dimers under the above-indicated conditions of storage and use.

The dimeric fraction contains antibodies with a higher specific activity than the monomeric fraction. Thus, this fraction is preferably used for the manufacture of a medicament against infections by a pathogen, e.g. viral, bacterial, fungal, protozoal and/or parasital infections. Especially preferred is the use of the dimeric fraction for the treatment of acute infections.

A further preferred application for dimeric fraction is a manufacture of a medicament against autoimmune disorders, e.g. and/or for the maintenance of autoimmune homeostasis.

Due to the high specific activity, the dimeric fraction may be administered in low doses resulting in higher compatibility and less side effects. Preferably, in this embodiment of the invention, the preparation is administered in a dose which is sufficient to obtain a therapeutic effect without causing undesired side effects.

The monomeric fraction on the other hand comprises natural antibodies which would be of particular benefit for the maintenance of immune homeostasis. A preferred application is the administration to children, senior persons or immunocompromised persons, e.g. persons suffering from disorders affecting the immune system or undergoing a therapy affecting the immune system. Especially preferred is the administration for anti-aging and/or rejuvenation therapy.

The dimer content of the monomer preparation is preferably limited to a maximum amount of 5%, preferably 2.5%, and more preferably 1 % in order to avoid adverse reactions for the treatment groups.

Still a further aspect of the present invention is a method for manufacturing pharmaceutical compositions comprising fractions of human antibodies or antigen-binding fragments thereof from a heterogenous human donor population comprising:
(a) subjecting a human antibody preparation from a heterogenous donor population to a size fractionation,
(b) recovering a fraction of dimeric antibodies and optionally monomerising the dimeric antibodies and
(c) recovering a fraction of monomeric antibodies.

The monomeric fraction is characterized by having a molecular weight of about 150 kDa in the size fractionation procedure. The dimeric fraction is characterized by having a molecular weight of about 300 kD in the size fractionation procedure.

Further, the invention shall be explained in more detail by the following Examples.

### Figures and Tables

### Figure 1

### Optimization of chromatography and fractionation of IVIG preparations

Various columns were tested as above using different flow rates (FR), different buffer systems and varying loading capacity for a 12% Sandoglobulin (SAGL) preparation. Fractions were collected and analysed by analytical HPLC for content of monomers, dimers and aggregates.

### Figure 2

### Preparative fractionation of IVIG preparations

A Sephacryl S-300 column (dimensions 26/60) was equilibrated in PBS/0.92% azide, followed by application of 500ml of a 12% Sandoglobulin (SAGL) preparation. The column was run at a flow rate of 1.2 ml/min and fractions 1-4 collected as indicated above and analysed by analytical HPLC for content of monomers, dimers and aggregates. This column allowed a scale up at the laboratory scale for preparation of monomer and dimer fractions.

### Figure 3

### Profiles of IVIG and separated monomeric and dimeric fractions on analytical HPLC

An analytical HPLC was used to analyse the % dimers, monomers and aggregates in the different fractions: IVIG freshly dissolved from a SAGL 12% preparation; monomeric and dimeric fractions isolated on a Sephacryl S-300 column.

### Figure 4

### Optimised flow chart for the size exclusion separation of monomers and dimers in IVIG

### Figure 5

### Kinetics of separated dimer fractions

The separated dimer fraction was kept at room temperature and analysed at intervals over a maximal time span of 48h, (range shown 0-24h) to observe the stability of the dimer fraction with respect to dissociation into monomers. Two separate experiments are shown. The straight line represents the % dimers in the dimeric fraction, the dotted line represents the % monomers in the dimeric fraction.

### Figure 6

### 2D PAGE analysis of separated monomer and dimer fractions

Two-dimensional polyacrylamide electrophoresis was performed as previously described in 2D-Electrophoresis, Principles and Methods (Amersham Biosciences). In brief, 4µg of total IVIG, monomeric and dimeric Immunoglobulin (Ig) fractions were diluted in 125µl of rehydration buffer containing 8M urea, 2% CHAPS, 0.002% bromphenol blue, DTT (0.65mM) and IPG Buffer 0.5%, each. 7cm IPG strips pH 3-10 (Amersham Biosciences), were rehydrated overnight and focused on an IPGphor unit (Amersham Biosciences), according to the strip specifications (step 1: constant 300V / 60min, step 2: gradient to 1000V / 30mins, step 3: gradient to 5000 V / 90min, step 4: constant 5000V / 15min). The focused strips were equilibrated in two steps of 15minutes in equilibration buffer containing 50mM Tris-HCl, pH 8.8, 6M urea, 30% (v/v) glycerol, 2% (v/v) SDS, 0.002% (v/v) bromphenol blue and either 10mg/ml DTT (1^{st} equilibration) or 25mg/ml iodoacetamide (2^{nd} equilibration). For the molecular weight separation, the strips were rinsed in sodium dodecyl sulfate (SDS) running buffer containing 2.5mM Tris (hydroxymethyl)-aminoethan (Merck), 25mM Glycin (Merck), 0.01 % SDS (w/v) (Flucka) and placed on a 12% polyacrylamide gel. Subsequently, the strips were overlayed with a 4% polyacrylamide stacking gel and the second dimension was run on a MiniProtean electrophoresis chamber (BioRad) at a constant 100V for approximately 90 minutes. Silver staining was performed using the PlusOne silver staining kit (Amersham Biosciences).

### Figure 7

### Reactivity to M proteins of S. pyogenes in IVIG fractions

ELISA: antigens (recombinant human M proteins) coated at 10µg/ml. Blocking PBS/casein 2hr 37°C. First antibody (IVIG T0/IVIG pH4.0/dimers/monomers) at 10, 100 and 1000µg/ml: 4hr at 37°C. Second antibody: AP004 (Binding Site) Sheep anti-human IgG Peroxidase, 1/1000 (2h, 37°C).

### Figure 8

### Differential activity of dimeric fraction on exoantigens Increased activity of dimeric fraction correlates with increase in affinity of binding

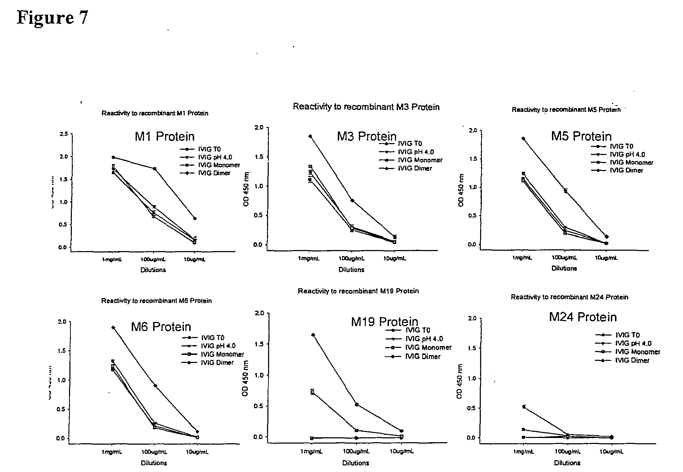

Affinity measurements against Tetanus toxoid and M1 protein. The affinities of different IVIG preparations were assessed by online monitoring using the IAsys cuvette system. The cuvettes were immobilized with 5 µg of either recombinant M1 protein from *Streptococcus pyogenes (S. pyogenes)* or Tetanus toxoid. To measure the association the IVIG preparations were diluted to different concentrations in PBS/0.05% Tween 20 and added in 50 µl samples to the cuvettes. The cuvettes were washed with 3 x 50 µl PBS/0.05% Tween 20 for monitoring the dissociation of antibodies. For regeneration the cuvettes were washed sequentially 3 times with 40 µl 20 mM HCl for 2 minutes and with 45 ul PBS/0.05% Tween 20. The K_{D} was calculated by plotting the kₒₙ values obtained for each concentration of IVIG, monomers or dimers using the FASTplot and the Grafit programs.
ELISA: IVIG, monomer and dimer fractions were analysed using commercial kits for Tetanus toxoid, respiratory syncytial virus (RSV) and H. *influenzae B* and the results expressed according to the units /g Immunoglobulin (Ig) calculated from a standard curve. Activity to the M1 protein was titrated out as described in Figure 7.

### Figure 9

### Dimers show increased activity on cytoskeletal proteins

### Immunofluorescence on HEp-2 cells

For immunohistology 50µl of IVIg, monomeric and dimeric Ig fractions were incubated on a diagnostic ANA (HEp-2 actin) slide (INOVA Diagnostics, Inc., San Diego, US) for 30minutes at 200µg/ml, followed by a 5 minute washing step in PBS. Subsequently, the FITC labelled anti-human IgG conjugate (INOVA Diagnostics, Inc., San Diego, US) was incubated for 20minutes, followed by another 5minute wash in PBS. Images were acquired at a 400x magnification on a fluorescence microscope (Nikon E600, Eclipse), employing the ACT-1 software (Nikon).

### Figure 10

### Dimers show increased activity on intracellular proteins of epithelial cells from different animal species

FACS analysis of epithelial cells from monkey (COS-7), human (HEP-G2, HEp-2) and hamster (CHO). Rat basophilic leukaemia cells wild type (RBL wt) were used as control non-epithelial cells.

For flow cytometry all adherent cell lines were detached with PBS-EDTA 0.03% (w/v), fixed with 1% (v/v) paraformaldehyde in PBS-casein 0.15% (w/v) (Sigma, Buchs, Switzerland) and permeabilized with 0.3% saponin (Sigma, Buchs, Switzerland) (w/v) in PBS-casein 0.15% (w/v). The indirect staining was performed in a duplicate multi-step procedure: (1) incubation of non-permeabilized or permeabilized cells for 20minutes with 50µg of IVIg, monomeric or dimeric Ig fractions in either PBS-casein 0.15% (w/v) or PBS-casein 0.15%/saponin 0.3% (both w/v), respectively. (2) Incubation with isotype specific FITC-labelled sheep anti-human IgG-FC (PFO04, The Binding Site, Birmingham) for 20minutes. Both incubations were followed with two 5 minute washing steps in PBS. Flow cytmetric data acquisition was done on a FACSCalibur machine using Cell Quest Pro software (both Becton-Dickinson), and for subsequent data analysis WinMDI software (©Joseph Trotter) was employed.

**Table 1 Differential reactivity of separated monomeric and dimeric fractions on selected antigens**

| ***Exoantigens*** | **Immunoglobulin fractions** | | ***Self-antigens*** | **Immunoglobulin fractions** | |
|---|---|---|---|---|---|
| | **Monomer s** | **Dimers** | | **Monomers** | **Dimers** |
| Tetanus toxoid^{ab} | **+** | **++** | Myosin^{b} | **+** | **++** |
| Diphtheria toxoid^{a} | **+** | **+** | Phosphorylase B^{b} | **+** | **++** |
| Campylobacter jejuni^{a} | **+** | **+** | Fibronectin^{b} | **-** | **-** |
| E. coli^{a} | **+** | **+/-** | α2-macroglobulin^{b} | **+** | **+** |
| Bacteriophage^{a} | **+** | **+** | C-reactive protein^{a} | **+** | **+** |
| KLH^{ab} | **+** | **+** | IgE, IgA, IgM^{a} | **+/-** | **+/-** |
| Bovine serum albumin^{a} | **+/-** | **+/-** | Human serum albumin^{a} | **-** | **-** |
| Streptavidin^{a} | **-** | **-** | Erythropoietin^{a} | **-** | **-** |
| | | | FcεRI-αchain^{a} | **+/-** | **+/-** |
| | | | C1q^{a} | **+** | **+** |

| | | | | | |
|---|---|---|---|---|---|
| Results summary from immunodot blots^{a} and ELISA^{b}. | | | | | |

**Immunodots:** air-dried dots with 10, 100 and 5.00 ng Antigen. Blocking: Top block, 1,5h, room temperature (RT). First antibody (dimers/monomers) at 10, 100 and 500µg/ml: over night at 4°C. Second antibody: AP004 (Binding Site) Sheep anti-human IgG Peroxidase (2h, RT).
Reactivities:
**+** strong reaction of 100 µg/ml immunoglobulin preparations on 10 ng antigen dots
**+/-** weak reactivity of 500 µg/ml immunoglobulin preparations on 500 ng antigen dots
- no reactivity of 500 µg/ml immunoglobulin preparations on 500 ng antigen dots
**ELISA:** antigens coated at 10, 1 µg/ml. Blocking PBS/casein 2hr 37°C. First antibody (dimers/monomers) at 10, 100 and 1000µg/ml: 4hr at 37°C. Second antibody: AP004 (Binding Site) Sheep anti-human IgG Peroxidase, 1/1000 (2h, 37°C).
Reactivities: comparison of reactivity at 10 µg/ml antigen with 100 µg/ml immunoglobulin fractions. **++** dimer signal titrates out at least logfold more than monomers, **+** clear signal over background, **+/-** weak signal but still above background, - no signal.

### Examples

We have investigated the differential reactivities of the monomeric and dimeric fractions found in IVIG preparations. We have used a lyophilized preparation which was solubilised according to the instructions for clinical *in vivo* use. This IVIG, also called SAGL , (short form for Sandoglobulin) was then aliquoted and stored at -20°C for further experiments. This IVIG was used as a reference in all further assays and to establish the conditions for separation of the monomeric and dimeric fractions. Our results have indicated a differential reactivity pattern of these fractions against both self/auto- and exo-antigens which has been confirmed using a variety of techniques.

### 1) Isolation of the monomeric and dimeric fractions of IVIG:

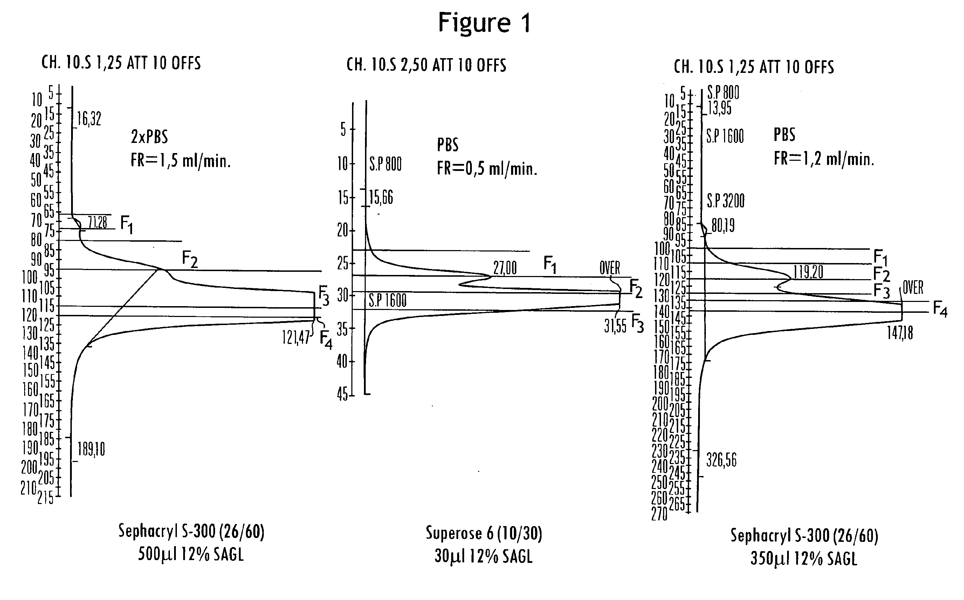

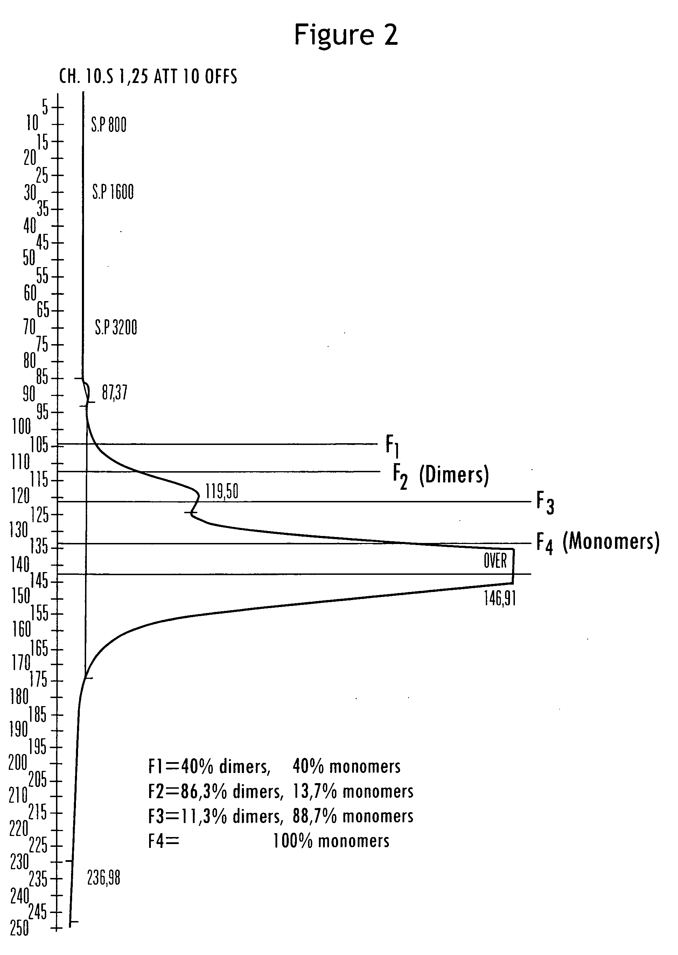

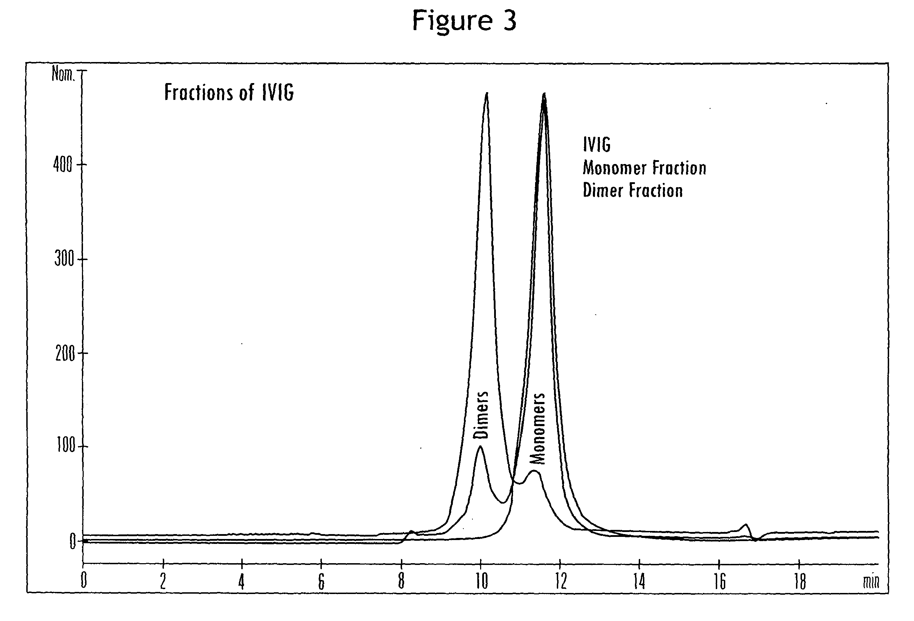

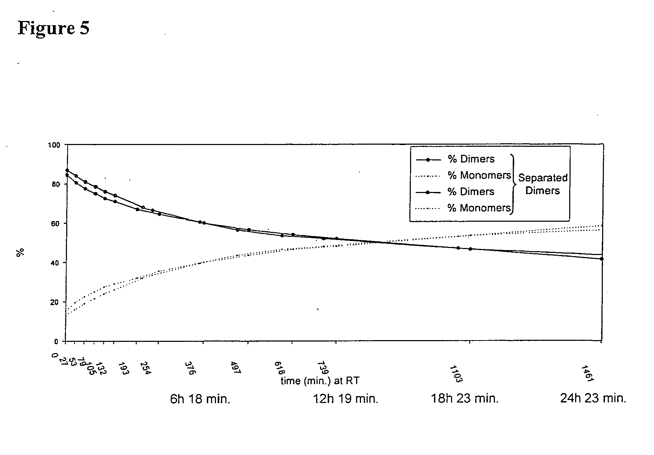

To optimize the fractionation we tested several different column types (Sephacryl, Superose, ReproSil) and different conditions (buffers, flow rates, size of columns). The Sephacryl S-300, a semi-preparative HPLC system, combined good peak separation, high purity of the fractions, short run times and high protein concentrations in the fractions, see Figures 1 and 2. The separated fractions were immediately analysed by HPLC, as shown in Figure 3. In agreement with previous reports we observed that dimers demonstrate a dynamic instability (ie. tend to revert to the monomeric form) influenced by buffer, pH, temperature, protein concentration and storage time. Figure 5 shows the kinetics of decay of the separated dimer fraction when stored at room temperature. The dimeric IgG reduced within hours to a more stable dimeric population, range 20-30% of the original dimers. In general, the formation of dimers is slow whereas their decay is faster. In contrast the monomeric fraction is stable over many weeks and does not reform dimers.

### 2) Preparation of a dissociated dimer preparation:

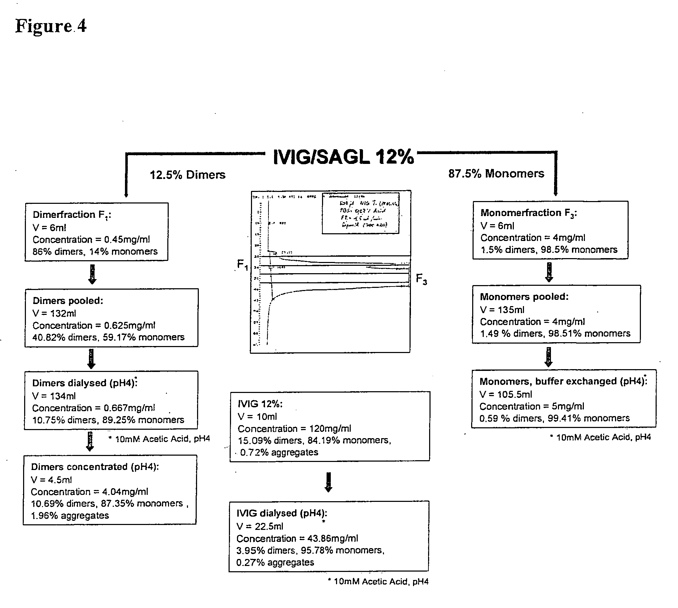

In order to produce a dimeric fraction that is dynamically stable and that would imitate the *in vivo* dissociation of dimers once injected a dissociated dimeric fraction was used which is effectively converted into its monomeric components. This means the dimeric fraction is separated by size exclusion followed by a treatment (in our eg. we used a dialysis into acetic acid at pH4.0 ) to convert the dimeric IgG into monomers. This required a minimum of 24h dialyis into 10mM acetic acid pH 4.0. Thereafter, this dissociated dimer preparation contained less than 5% dimers, remained dynamically stable and does not re-convert to dimers under defined conditions of storage and use. Further, neutralization of the dissociated dimers to pH 7.4 in PBS buffer had no effect on the % dimers after up to 24h storage even at room temperature. Thus for the duration of the assay conditions used to assess reactivity, the dissociated dimers would remain dissociated. To allow equal comparison of monomers and dimers the monomers were also subjected to the same treatment and compared in the same buffer system. An example of an optimized flow chart for the separation and preparation of the various fractions used is shown in Figure 4.

### 3) Characterisation of the monomeric and dissociated dimer preparations

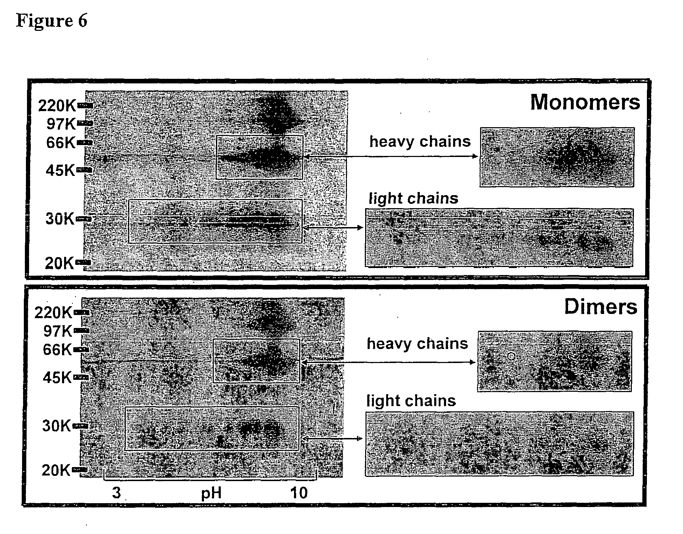

Both fractions demonstrated the expected overall patterns for heavy and light chains on 2D gel electrophoresis. The dimeric fraction showed a tendency for a wider isoelectric distribution extending more into the acidic region, Figure 6. Comparative isotype analysis showed equal distribution of IgG1, 2, 4 and slightly increased IgG3 in the dimeric fractions.

### 4) Differential reactivities of monomeric and dissociated dimer preparations

Reactivities to both exo- and self/auto- antigens in a range of different assays have been performed. In summary, the dimeric fraction shows an increased reactivity to certain antigens both self antigens and exoantigens. In some cases we have also measured the affinity of the reaction which was clearly increased in the dimers. We have not found an antigen specificity where the reactivity is only present in either the monomers or the dimers. There are antigens where no detectable antibody reactivity was detected in either the monomers or the dimers.

Figure 7 shows results from an ELISA using human recombinant M proteins (virulence factors) of *Streptococcus pyogenes.* The dimers show a clearly enhanced reaction compared to either the monomers or the IVIG preparations defined as T0 (IVIG prepared and frozen at Time zero) or IVIG defined as pH4.0 (IVIG dialysed into the same buffer as the dimers).

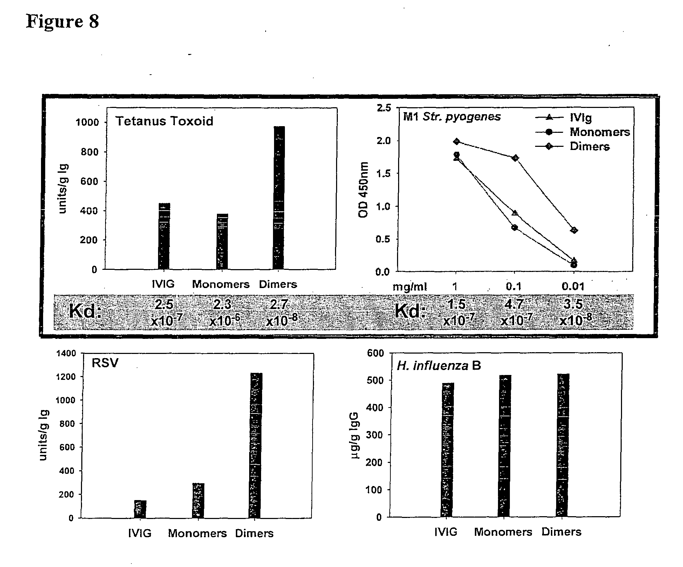

Figure 8 shows results for the exoantigens Tetanus toxoid, M1 of S. *pyogenes ,* RSV and H. influenzae B. The dimers show clearly enhanced reactions, confirmed by affinity measurements except for H. influenzae B which shows equal reactivity in both fractions.

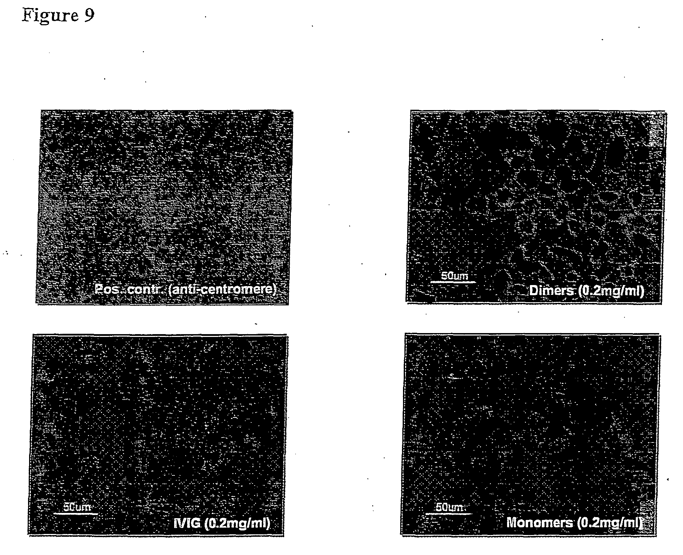

Figure 9 shows results using the HEp-2 cells for immunohistological analysis. These cells are routinely used by diagnostic laboratories to detect autoantibodies, found in various autoimmune diseases reacting with a range of nuclear, ribosomal and mitochondrial antigens. No pathological pattern of reactivity was seen. Instead there was a marked activity of the dimeric fraction which at higher magnification looked like cytoskeletal components of the cell.

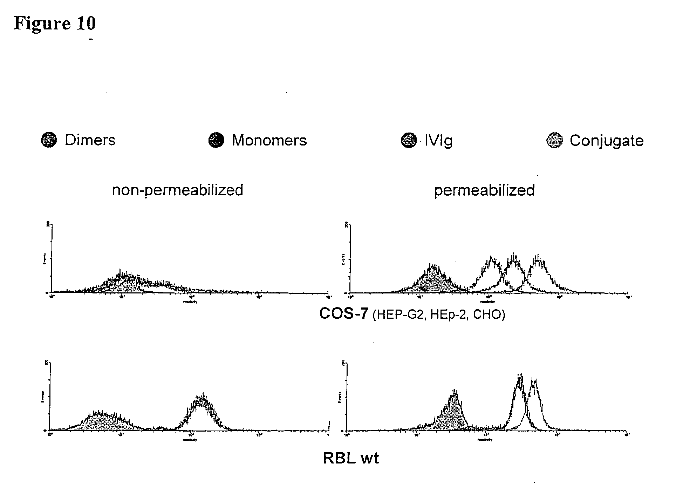

Figure 10 shows results from FACS analysis on different types of epithelial cells and rat basophil leukaemia (RBL) cells. Cells were either not treated or treated ie. permeabilized to allow distinction between membrane and intracellular staining respectively. The dimers show increased intracellular reactivity for all the epithelial cells tested. No significant staining over background was seen for the non-permeabilized cells indicating a lack of reactivity with membrane proteins for all fractions tested. In contrast , all fractions demonstrated equal staining on the non-treated RBL cells probably representing interaction with Fcgamma receptors. There was a small increase in dimer reactivity compared to the other fractions on permeabilized cells.

Table 1 represents cumulative data from both ELISA and Immunodot assays where the respective antigens are immobilized and allowed to react with a concentration range of monomers ,dimers and IVIG start preparation as control. To allow a convenient comparison the monomers and dimers have been compared on a simple reactivity scale (++ to -). Differential reactivities are clearly seen between the monomers and dimmers. It also seems that some self antigens eg. myosin and phosphorylase B, while reacting with the monomers also show an increased reactivity with the dimers.

### Summary

Based on the above results, the dimeric fraction contains the more mature antibody repertoire in contrast to the monomeric fraction which essentially consists of natural antibodies.

The dimeric fraction contains higher affinity antibodies and thus this fraction would also be biologically more active eg. neutralizing activity in the case of anti- bacterial, -toxin or -viral antibodies. Thus the dimeric fraction represents a sub-fraction of IVIG with a higher specific activity to certain antigens. This would also account for the presence of anti-idiotype antibodies in this fraction as the immune system has to respond to the high affinity antibodies being produced by a negative feedback or down regulation to maintain homeostasis.

The monomeric fraction contains the lower affinity antibodies providing the natural antibodies ie. the basic repertoire from which the high affinity antibodies must develop.

### References

1. Lacroix-Desmazes, S., et al., *Analysis of the natural human IgG antibody repertoire: life-long stability of reactivities towards self antigens contrasts with age-dependent diversification of reactivities against bacterial antigens.* Eur J Immunol, 1995. 25(9): p. 2598-604.
2. Kaveri, S., et al., *Antibodies to a conserved region of HLA class I molecules, capable of modulating CD8 T cell-mediated function, are present in pooled normal immunoglobulin for therapeutic use.* J Clin Invest, 1996. 97(3): p. 865-9.
3. Bouhlal, H., et al., *Antibodies to C-C chemokine receptor 5 in normal human IgG block infection of macrophages and lymphocytes with primary R5-tropic strains of HIV-1.* J Immunol, 2001. 166(12): p. 7606-11.
4. Vassilev, T., et al., *Antibodies to the CD5 molecule in normal human immunoglobulins for therapeutic use (intravenous immunoglobulins, IVIg).* Clin Exp Immunol, 1993. 92(3): p. 369-72.
5. Hurez, V., et al., *Anti-CD4 activity of normal human immunoglobulin G for therapeutic use. (Intravenous immunoglobulin, IVIg).* Ther Immunol, 1994. 1(5): p. 269-77.
6. Rossi, F., G. Dietrich, and M.D. Kazatchkine, *Anti-idiotypes against autoantibodies in normal immunoglobulins: evidence for network regulation of human autoimmune responses.* Immunol Rev, 1989. 110: p. 135-49.
7. Rossi, F. and M.D. Kazatchkine, *Antiidiotypes against autoantibodies in pooled normal human polyspecihc Ig.* J Immunol, 1989. 143(12): p. 4104-9.
8. Stollar, B.D., *Autoantibodies and autoantigens: a conserved system that may shape a primary immunoglobulin gene pool.* Mol Immunol, 1991. 28(12): p. 1399-412.
9. Pashov, A., et al., *Autoantibodies to heat shock protein 90 in the human natural antibody repertoire.* Int Immunol, 2002. 14(5): p. 453-61.
10. Dalakas, M.C., *Blockade of blocking antibodies in Guillain-Barre syndromes: "unblocking" the mystery of action of intravenous immunoglobulin.* Ann Neurol, 2002. 51(6): p. 667-9.
11. Jacobs, B.C., P.I. Schmitz, and F.G. van der Meche, *Campylobactery jejuni Infection and treatment for Guillain-Barre Syndrome.* N Engl J Med, 1996. 335(3): p. 208-9.
12. Ginaldi, L., et al., *Cell proliferation and apoptosis in the immune system in the elderly.* Immunol Res, 2000. 21(1): p. 31-8.
13. Ginaldi, L., et al., *Changes in the expression of surface receptors on lymphocyte subsets in the elderly: quantitative flow cytometric analysis.* Am J Hematol, 2001. 67(2): p. 63-72.
14. Yuki, N., et al., *Clinical features and response to treatment in Guillain-Barre syndrome associated with antibodies to GM1b ganglioside.* Ann Neurol, 2000. 47(3): p. 314-21.
15. Cohen, I.R., *The cognitive paradigm and the immunological homunculus.* Immunol Today, 1992. 13(12): p. 490-4.
16. Altznauer, F., et al., *Concurrent presence of agonistic and antagonistic anti-CD95 autoantibodies in intravenous Ig preparations.* J Allergy Clin Immunol, 2003. 112(6): p. 1185-90.
17. Ahmed, A.R. and M.V. Dahl, Consensus *statement on the use of intravenous immunoglobulin therapy in the treatment of autoimmune mucocutaneous blistering diseases.* Arch Dermatol, 2003. 139(8): p. 1051-9.
18. Webster, A.D., et al., *Critical dependence on antibody for defence against mycoplasmas.* Clin Exp Immunol, 1988. 71(3): p. 383-7.
19. Jolles, S., J. Hughes, and S. Whittaker, *Dermatological uses of high-dose intravenous immunoglobulin.* Arch Dermatol, 1998. 134(1): p. 80-6.
20. Tankersley, D.L., *Dimer formation in immunoglobulin preparations and speculations on the mechanism of action of intravenous immune globulin in autoimmune diseases.* Immunol Rev, 1994. 139: p. 159-72.
21. Nobrega, A., et al., *Global analysis of antibody repertoires. II. Evidence for specificity, self-selection and the immunological "homunculus" of antibodies in normal serum.* Eur J Immunol, 1993. 23 (11): p. 2851-9.
22. Berger, A.R., et al., *Guidelines for the diagnosis and treatment of chronic inflammatory demyelinating polyneuropathy.* J Peripher Nerv Syst, 2003. 8(4): p. 282-4.
23. Imbach, P., et al., *High-dose intravenous gammaglobulin for idiopathic thrombocytopenic purpura in childhood.* Lancet, 1981. 1(8232): p. 1228-31.
24. Dalakas, M.C., et al., *High-dose intravenous immune globulin for stiff-person syndrome.* N Engl J Med, 2001. 345(26): p. 1870-6.
25. Jolles, S., *High-dose intravenous immunoglobulin (hdlVlg) in the treatment of autoimmune blistering disorders.* Clin Exp Immunol, 2002. 129(3): p. 385-9.
26. Dalakas, M.C., *High-dose intravenous immunoglobulin in inflammatory myopathies: experience* based on *controlled clinical trials.* Neurol Sci, 2003. 24 Suppl 4: p. S256-9.
27. Ryan, A., B.J. Thomson, and A.D. Webster, *Home intravenous immunoglobulin therapy for patients with primary hypogammaglobulinaemia.* Lancet, 1988. 2(8614): p. 793.
28. Kieseier, B.C., M.C. Dalakas, and H.P. Hartung, *Immune mechanisms in chronic inflammatory demyelinating neuropathy.* Neurology, 2002. 59(12 Suppl 6): p. S7-12.
29. Ginaldi, L., et al., *The immune system in the elderly: activation-induced and damage-induced apoptosis.* Immunol Res, 2004. 30(1): p. 81-94.
30. Ginaldi, L., et al., *The immune system in the elderly: I. Specific humoral immunity.* Immunol Res, 1999. 20(2): p. 101-8.
31. Ginaldi, L., et al., *The immune system in the elderly: II. Specific cellular immunity.* Immunol Res, 1999. 20(2): p. 109-15.
32. Ginaldi, L., et al., *The immune system in the elderly: III. Innate immunity.* Immunol Res, 1999. 20(2): p. 117-26.
33. Roux, K.H. and D.W. Metzger, *Immunoelectron microscopic localization of idiotypes and allotypes on immunoglobulin molecules.* J Immunol, 1982. 129(6): p. 2548-53.
34. Ichiyama, T., et al., *An immunoglobulin agent (IVIG) inhibits NF-kappaB activation in cultured endothelial cells of coronary arteries in vitro.* Inflamm Res, 2004. 53(6): p. 253-6.
35. Tankersley, D.L., M.S. Preston, and J.S. Finlayson, *Immunoglobulin G dimer: an idiotype-anti-idiotype complex.* Mol Immunol, 1988. 25(1): p. 41-8.
36. Kazatchkine, M.D. and S.V. Kaveri, *Immunomodulation of autoimmune and inflammatory diseases with intravenous immune globulin.* N Engl J Med, 2001. 345(10): p. 747-55.
37. Sewell, W.A. and S. Jolles, *Immunomodulatory action of intravenous immunoglobulin.* Immunology, 2002. 107(4): p. 387-93.
38. Ginaldi, L., et al., *Immunosenescence and infectious diseases.* Microbes Infect, 2001. 3(10): p. 851-7.
39. Gold, R., M.C. Dalakas, and K.V. Toyka, *Immunotherapy in autoimmune neuromuscular disorders.* Lancet Neurol, 2003. 2(1): p. 22-32.
40. van der Meche, F.G., P.A. van Doorn, and B.C. Jacobs, *Inflammatory neuropathies--pathogenesis and the role of intravenous immune globulin.* J Clin Immunol, 1995. 15(6 Suppl): p. 63S-69S.
41. Sami, N., et al., *Influence of intravenous immunoglobulin therapy on autoantibody titres to BP Ag1 and BP Ag2 in patients with bullous pemphigoid.* J Eur Acad Dermatol Venereol, 2003. 17(6): p. 641-5.
42. Bayry, J., et al., *Inhibition of maturation and function of dendritic cells by intravenous immunoglobulin.* Blood, 2003. 101 (2): p. 758-65.
43. So, A., et al., *Intravenous gammaglobulin treatment in patients with hypogammaglobulinaemia.* Br Med J (Clin Res Ed), 1984. 289(6453): p. 1177-8.
44. Bayry, J., et al., *Intravenous immunoglobulin for infectious diseases: back to the pre-antibiotic and passive prophylaxis era?* Trends Pharmacol Sci, 2004. 25(6): p. 306-10.
45. Dalakas, M.C., *Intravenous immunoglobulin in autoimmune neuromuscular diseases.* Jama, 2004. 291 (19): p. 2367-75.
46. Teeling, J.L., et al., *Intravenous immunoglobulin preparations induce mild activation of neutrophils in vivo via triggering of macrophagesstudies in* a *rat model.* Br J Haematol, 2001. 112(4): p. 1031-40.
47. Ahmed, A.R., *Intravenous immunoglobulin therapy in the treatment of patients with pemphigus vulgaris unresponsive to conventional immunosuppressive treatment.* J Am Acad Dermatol, 2001. 45(5): p. 679-90.
48. Webster, A.D., *Intravenous immunoglobulins.* Bmj, 1991. 303(6799): p. 375-6.
49. Schussler, O., et al., *Intravenous immunoglobulins for therapeutic use contain anti-idiotypes against xenophile antibodies and prolong discordant graft survival.* Clin Immunol Immunopathol, 1998. 86(2): p. 183-91.
50. de Grandmont, M.J., et al., *Intravenous immunoglobulins induce the in vitro differentiation* of *human B lymphocytes and the secretion of IgG.* Blood, 2003. 101(8): p. 3065-73.
51. Simon, H.U. and P.J. Spath, *IVIG--mechanisms of action.* Allergy, 2003. 58(7): p. 543-52.
52. Hany, M., A. Ryan, and A.D. Webster, *Long-term safety and tolerability* of *intravenous immunoglobulin.* Lancet, 1991. 338(8760): p. 194-5.
53. Dalakas, M.C., *Mechanisms of action of IVIg and therapeutic considerations in the treatment of acute and chronic demyelinating neuropathies.* Neurology, 2002. 59(12 Suppl 6): p. S13-21.
54. Xu, C., et al., *Modulation of endothelial cell function by normal polyspecific human intravenous immunoglobulins:* a *possible mechanism* of *action in vascular diseases.* Am J Pathol, 1998. 153(4): p. 1257-66.
55. Lacroix-Desmazes, S., et al., *Natural antibodies to factor VIII.* Semin Thromb Hemost, 2000. 26(2): p. 157-65.
56. Coutinho, A., M:D. Kazatchkine, and S. Avrameas, *Natural autoantibodies.* Curr Opin Immunol, 1995. 7(6): p. 812-8.
57. Hurez, V., et al., *Polyreactivity is* a *property of natural and disease-associated human autoantibodies.* Scand J Immunol, 1993. 38(2): p. 190-6.
58. Dietrich, G., et al., *Selection of the expressed B cell repertoire by infusion of normal immunoglobulin G in a patient with autoimmune thyroiditis.* Eur J Immunol, 1993. 23(11): p. 2945-50.
59. Lacroix-Desmazes, S., et al., *Self-reactive antibodies (natural autoantibodies) in healthy individuals.* J Immunol Methods, 1998. 216 (1-2): p. 117-37.
60. Lacroix-Desmazes, S., et al., *Stability of natural self-reactive antibody repertoires during aging.* J Clin Immunol, 1999. 19(1): p. 26-34.
61. Ahmed, A.R., *Treatment of autoimmune mucocutaneous blistering diseases with intravenous immunoglobulin therapy.* Expert Opin Investig Drugs, 2004. 13(8): p. 1019-32.
62. Sami, N., K.C. Bhol, and A.R. Ahmed, *Treatment of oral pemphigoid with intravenous immunoglobulin* as *monotherapy. Long-term follow-up: influence of treatment on antibody titres to human alpha6 integrin.* Clin Exp Immunol, 2002. 129(3): p. 533-40.
63. Dalakas, M.C., *The use* of *intravenous immunoglobulin in the treatment of autoimmune neuromuscular diseases: evidence-based indications and safety profile.* Pharmacol Ther, 2004. 102(3): p. 177-93.
64. Vassilev, T.L., et al., *Variable region-connected, dimeric fraction of intravenous immunoglobulin enriched in natural autoantibodies.* J Autoimmun, 1995. 8(3): p. 405-13.
65. Roux, K.H. and D.L. Tankersley, *A view of the human idiotypic repertoire. Electron microscopic and immunologic analyses of spontaneous idiotype-anti-idiotype dimers in pooled human IgG.* J Immunol, 1990. 144(4): p. 1387-95.

## Claims

1. A pharmaceutical composition comprising a fraction of human antibodies or antigen-binding fragments thereof from a heterogenous human donor population wherein the fraction originates from the dimeric human antibodies.

2. The composition of claim 1 wherein the fraction of human antibodies is a fraction of dimeric antibodies from a polyclonal human immunoglobulin preparation, preferably from an intravenous immunoglobulin preparation.

3. The composition of claim 1 or 2 wherein the purity of dimeric antibodies is at least 75%, preferably at least 80% and more preferably at least 85%.

4. The composition of any one of claims 1-3 wherein the human antibodies have at least a two fold reactivity over the non-fractionated immunoglobulin preparation.

5. The composition of any one of claims 1-4 wherein the dimeric fraction of human antibodies is obtainable by size fractionation of human antibody preparations from a heterogenous donor population and recovering antibody dimers with an apparent molecular weight of about 300 kD.

6. The composition of claim 5 wherein the size fractionation comprises size exclusion, separation, gel filtration, ultrafiltration and/or other gel or membrane separation methods.

7. The compositions of claims 1-6 wherein the dimeric antibodies or antibody fragments are present in a dissociated or monomerised form.

8. The use of a pharmaceutical composition of any one of claims 1-7 for the manufacture of a medicament against infectious, e.g. viral, bacterial, fungal, protozoal or parasital infections.

9. The use of claim 8 for the treatment of acute infections.

10. The use of a pharmaceutical composition of any one of claims 1-7 for the manufacture of a medicament against autoimmune disorders.

11. The use of claim 10 for the maintenance and/or restoration of autoimmune homeostasis.

12. A pharmaceutical composition comprising a fraction of human antibodies or antigen-binding fragments thereof from a heterogenous human donor population wherein the fraction originates from the monomeric human antibodies.

13. The composition of claim 12 wherein the fraction of human antibodies is a fraction of monomeric antibodies from a polyclonal human immunoglobulin preparation, preferably from an intravenous immunoglobulin preparation.

14. The composition of claim 12 or 13 wherein the purity of monomeric antibodies is at least 85%, preferably at least 90% and more preferably at least 95%.

15. The composition of any one of claims 12-14 wherein the fraction of monomeric human antibodies is obtainable by size fractionation of human antibody preparations from a heterogenous donor population and recovering antibody monomers with an apparant molecular weight of about 150 kD.

16. The composition of claim 15 wherein the size fractionation comprises size exclusion separation, gel filtration, ultrafiltration and/or other gel or membrane separation methods.

17. The use of a pharmaceutical composition of any one of claims 12-16 for the manufacture of a medicament for the maintenance of immune homeostasis.

18. The use of claim 17 for the administration to children, to senior persons or immunocompromised persons, e.g. persons suffering from disorders affecting the immune system or undergoing a therapy affecting the immune system.

19. The use of claim 18 for an anti-aging or rejuvenation therapy.

20. A method for manufacturing pharmaceutical compositions comprising fractions of human antibodies or antigen-binding fragments thereof from a heterogenous human donor population comprising:
(a) subjecting a human antibody preparation from a heterogenous donor population to a size fractionation,
(b) recovering a fraction of dimeric antibodies and optionally monomerising the dimeric antibodies and
(c) recovering a fraction of monomeric antibodies.

21. The method of claim 20 wherein the fraction of monomeric human antibodies is **characterized in** having an apparent molecular weight of about 150 kDa and the fraction of dimeric antibodies is **characterized in** having an apparent molecular weight of about 300 kDa.

22. The method of claim 20 or 21 wherein fractions (b) and (c) are manufactured for different pharmaceutical applications.
